# FASCICULE DE BREVET EUROPEEN

(11) **EP 1 414 358 B1**
(45) Date de publication et mention de la délivrance du brevet: **30.12.2015**
(21) Numéro de dépôt: 02772494.7
(22) Date de dépôt: 08.08.2002
(51) Int. Cl.: A61F 2/44, A61F 2/46, A61B 17/70, A61F 2/30

(54) **ENSEMBLE DE STABILISATION DE VERTEBRES**
ANORDNUNG ZUR STABILISIERUNG DES RÜCKGRATES
VERTEBRA STABILIZING ASSEMBLY

(30) Priorité: 08.08.2001 FR 0110604
(43) Date de publication de la demande: 06.05.2004
(73) Titulaire: Kyphon SÀRL, 2000 Neuchâtel (CH)
(72) Inventeur: TAYLOR, Jean, 06400 Cannes (FR)
(74) Mandataire: Price, Nigel John King
(86) Numéro de dépôt international: PCT/FR2002/002834
(87) Numéro de publication internationale: WO 2003/015646

(56) Documents cités:
- EP-A- 0 743 045
- WO-A-00/53126
- DE-C- 3 922 203
- FR-A- 2 775 183
- US-A- 5 716 416
- PATENT ABSTRACTS OF JAPAN vol. 017, no. 647 (C-1135), 2 décembre 1993 (1993-12-02) & JP 05 208029 A (KYOCERA CORP), 20 août 1993 (1993-08-20)

## Description

La présente invention concerne un ensemble de stabilisation de vertèbres, particulièrement de vertèbres lombaires. Cet ensemble peut être défini comme une prothèse d'assistance à effet couplé, comme cela apparaîtra plus loin.

On sait que la mobilité de deux vertèbres adjacentes, particulièrement dans le secteur lombaire, est sous la dépendance du disque intervertébral sur le côté antérieur et des paires de facettes articulaires sur le côté postérieur.

La disposition du disque et des facettes articulaires assure une autolimitation des mouvements, tant dans le plan perpendiculaire à la colonne vertébrale, lors des torsions, que dans un plan sagittal, lors de flexions et extensions de la colonne vertébrale.

La forme, la structure et la hauteur du disque, confèrent à ce dernier des fonctions complémentaires de maintien de lordose, d'amortissement, et de répartition des contraintes. En arrière, les facettes articulaires guident les mouvements et jouent le rôle de charnières.

Le processus de vieillissement du sujet amène des perturbations de cet équilibre disco-facettaire antéro-postérieur. Généralement, la dégénérescence discale précède celle des facettes. Le collapsus et l'instabilité discale conduisent à un report partiel des contraintes au niveau des colonnes postérieures formées par les facettes, qui provoque une perte de la congruence facettaire et un relâchement ligamentaire. Il en résulte une détérioration des surfaces articulaires, qui conduisent à diverses pathologies ayant des implications à caractère mécanique et neurologique.

Pour traiter ces pathologies, il a été envisagé de procéder à une arthrodèse antérieure par insertion d'un greffon entre les plateaux des deux vertèbres concernées. Ce greffon est la plupart du temps contenu dans une cage rigide, dite "cage de fusion".

Cette technique laisse toutefois persister une instabilité rotatoire susceptible de compromettre à moyen terme le résultat antalgique escompté, et il est maintenant établi que l'arthrodèse antérieure doit être complétée par une arthrodèse postérieure.

Une intervention de ce type présente des limites et des inconvénients. En effet, elle s'adresse à des pathologies sévères se trouvant à un stade avancé, et n'est pas dénuée de risques étant donné que les patients sont souvent pris en charge à un stade relativement tardif de l'évolution pathologique. De plus, elle peut avoir des conséquences néfastes au niveau des articulations adjacentes, à moyen et long terme.

Pour ces raisons, des techniques dites "de non-fusion" se sont développées, s'intéressant au traitement précoce et palliatif des phénomènes dégénératifs disco-facettaires.

Concernant l'espace discal, il a été développé différents implants amortisseurs visant à remplacer le *nucleus pulposus,* sous forme de paires de coussins, ou d'éléments elliptiques ou en spirale.

L'introduction de ces implants se fait soit par un accès antérieur, ce qui a pour inconvénient de léser le ligament vertébral commun antérieur, soit par un accès postérieur, ce qui a pour inconvénient de conduire à des sacrifices osseux non négligeables, du fait de l'encombrement des implants.

Des techniques d'injection percutanée d'un colloïde susceptible de se polymériser *in situ* ont également été envisagées, de même que des techniques utilisant des prothèses discales massives sous la forme de deux plaques métalliques enserrant une matière amortissante et venant prendre appui au niveau de chacun des plateaux vertébraux adjacents au disque.

Ces techniques ne donnent pas entièrement satisfaction en ce qui concerne le traitement d'une dégénérescence discale combinée à une usure facettaire et/ou à une distension ligamentaire.

Des dispositifs visant à limiter le jeu articulaire des facettes ont également été envisagés. Un dispositif de ce type comprend notamment un ligament continu tressé, mis en place entre les épineuses ou le long des facettes articulaires par le biais de vis pédiculaires, et/ou un élément distractant amortissant, mis en place à la jonction lame-épineuse de manière à soulager le jeu facettaire tout en retendant les éléments capsuloligamentaires postérieurs.

Il s'avère que ces techniques ne donnent également pas entièrement satisfaction en ce qui concerne le traitement précité.

La présente invention vise à remédier à cet inconvénient fondamental, en fournissant un ensemble de stabilisation de deux vertèbres adjacentes subissant à la fois une dégénérescence discale avec une distension ligamentaire, ainsi qu'éventuellement une usure des facettes, cet ensemble devant en outre être relativement simple à implanter et être par ailleurs relativement peu invasif.

EP 0 743 045 A2 divulgue un dispositif pour l'ostéosynthèse et de nature à être préparé avec une matière qui présente un effet de mémoire de forme. Le dispositif est fait de fil et contient une partie opérationnelle et des composants de verrouillage. Les composants de verrouillage sont comme des jambes de plongée. La partie opérationnelle consiste à alterner des éléments arqués, avec une longueur d'arc dépassant 180 degrés. Les composants adjacents sont placés aux différents côtés de la ligne reliant leurs points de flexion aux jambes de plongée. L'une des jambes de plongée est fournie avec un élément arqué de la forme de la surface de l'os adjacent et a une longueur de 1,5 à 2,0 fois supérieure à celle de l'autre jambe.

Selon la présente invention, il s'agit d'un ensemble de stabilisation de vertèbres lombaires, l'ensemble étant tel que revendiqué dans la revendication 1 annexée.

Des supplémentaires de l'ensemble sont exposés dans les revendications 2-15.

L'ensemble selon l'invention permet ainsi non seulement de rétablir l'écartement anatomique des vertèbres tant entre les facettes qu'entre les plateaux vertébraux, mais également, et surtout, en conservant le ligament commun antérieur et le ligament postérieur supra-épineux, de remettre ces ligaments en tension anatomique.

Cette mise en tension a pour effet de restaurer la "balance" ligamentaire anatomique existant entre ces ligaments tout en rendant au disque et aux facettes leurs fonctions anatomiques, à savoir la fonction d'amortissement en ce qui concerne le disque et les fonctions de charnière et d'équilibrage postérieur en ce qui concerne les facettes.

L'implant postérieur est mis en place immédiatement en arrière des facettes, en lieu et place du complexe ligamentaire inter-épineux, et est susceptible d'être sollicité tant en compression qu'en étirement.

L'implant inter-corporéal est quant à lui positionné de préférence le plus en avant possible, le long du pourtour des plateaux vertébraux. Il se trouve ainsi placé là où les contraintes sont maximales. Son éloignement maximal de l'implant postérieur permet de rétablir au mieux ladite balance ligamentaire antéro-postérieure.

L'implant postérieur et l'implant inter-corporéal ont ainsi pour fonction d'absorber les contraintes, tant en compression qu'en élongation, générées lors des mouvements de flexion de la colonne vertébrale vers l'avant et vers l'arrière. Lors d'une flexion de la colonne vertébrale vers l'avant, le ligament postérieur supra-épineux, remis en tension fonctionnelle par l'implant postérieur, assure sa fonction anatomique de limitation du mouvement, optimisée et renforcée grâce à l'assistance et au contrôle opéré par l'implant postérieur, du fait de l'étirement progressif limitant de celui-ci. Simultanément, l'implant inter-corporéal apporte une assistance au disque défaillant en absorbant les contraintes exercées en pression par le corps vertébral supérieur sur le corps vertébral inférieur et atténue le phénomène dit de "creep", c'est-à-dire l'enfoncement du disque sous l'exercice d'une pression. Cet implant inter-corporéal fournit conjointement une assistance limitante qui limite l'effet d'étirement subit par les structures souples postérieures. Lors d'une flexion de la colonne vertébrale vers l'arrière, le ligament vertébral antérieur, remis en tension fonctionnelle par l'implant inter-corporéal, assure sa fonction anatomique de limitation progressive du mouvement, assistée et encadrée par l'implant amortissant étiré. Simultanément, l'implant postérieur est comprimé et optimise le jeu des facettes dans leur fonction de charnière et d'équilibrage postérieur.

L'action de chaque implant se combine par conséquent à l'action de l'autre implant. À l'étirement de l'un des implants répond une compression de l'autre implant, par un effet d'autolimitation interdépendante.

L'utilisation d'une voie d'abord unique permet en outre de simplifier grandement l'intervention et de rendre l'ensemble selon l'invention très peu invasif.

Des moyens de maintien sont avantageusement prévus pour assurer la continuité de l'implant postérieur en position, avec les apophyses épineuses. Ces moyens de maintien peuvent comprendre une conformation appropriée de l'implant postérieur, définissant des évidements opposés de réception des apophyses épineuses et conférant à l'implant postérieur une forme "en diabolo" ou en "H", et/ou des moyens de fixation de l'implant postérieur aux apophyses épineuses, tels que deux liens indépendants ou des pièces rigides d'ancrage ne limitant pas la déformabilité de l'implant.

L'implant postérieur peut être constitué en une seule pièce ou peut être en deux parties assemblables, amenées chacune par un côté de l'espace inter-épineux et assemblées l'une à l'autre au niveau de cet espace. Lorsque cet implant postérieur a une forme "en diabolo" ou en "H" et qu'il est en une seule pièce, l'ensemble peut comprendre une pièce venant maintenir cet implant dans un état déformé dans lequel deux oreilles latérales que comprend l'implant sont rapprochées l'une de l'autre, de manière à permettre une insertion latérale de l'implant entre les apophyses épineuses des vertèbres traitées.

L'ensemble selon l'invention peut comprendre un implant intercorporéal conformé de manière à s'étendre au niveau des zones latérales antérieures des plateaux vertébraux, afin de renforcer la stabilité latérale des vertèbres et de permettre une assistance de *l'annulus fibrosus* périphérique ; il peut alors notamment présenter une forme courbe, en portion d'anneau.

L'ensemble selon l'invention peut également comprendre, en remplacement ou en complément d'un implant inter-corporéal d'assistance de l'*annulus fibrosus* périphérique, un implant inter-corporéal prévu pour remplacer et/ou assister le *nucléus.* Cet implant peut alors présenter une forme générale en "haricot" ou en "oméga", avec une portion médiane prolongée par deux lobes latéraux faisant saillie sur le côté postérieur. Il peut également présenter une forme en sphère.

De préférence, au moins un implant inter-corporéal a une section transversale triangulaire ou trapézoïdale, et est destiné à être implanté avec son plus grand côté latéral tourné vers le côté antérieur.

Cet implant, ainsi conformé, respecte la pente discale anatomique.

Au moins un implant inter-corporéal peut comprendre des moyens pour assurer son maintien entre les plateaux vertébraux. Notamment, ces moyens comprennent une conformation de l'implant inter-corporéal adaptée à la forme des plateaux vertébraux, propre à maintenir cet implant inter-corporéal entre ces plateaux. Lorsque l'implant inter-corporéal est en forme de sphère, il peut comprendre un rebord équatorial réduisant son risque de déplacement.

L'implant postérieur peut comprendre un noyau en matériau amortissant, tel qu'un silicone, un polyuréthane, un polymère hydrophile, un polycarbonate, ou une pièce en métal à mémoire de forme, et une enveloppe entourant ce noyau. Cette enveloppe permet de protéger utilement ce noyau ou cette pièce contre les frottements. L'enveloppe peut être formée de fibres tressées.

L'implant inter-corporéal peut avoir une structure identique.

La mise en place de cet implant inter-corporéal peut notamment être réalisée au moyen d'un tube-guide introducteur pourvu d'un piston, cet implant étant engagé, en étant comprimé, dans ce tube-introducteur et pouvant être expulsé de ce dernier au moyen du piston.

Le tube-introducteur comprime transitoirement l'implant. Cette réduction de volume, c'est-à-dire de l'encombrement de l'implant, permet de se contenter d'un accès chirurgical analogue à celui d'une dissectomie, évitant tout sacrifice osseux déstabilisant. Le tube est introduit dans l'espace discal par voie postéro-latérale transligamentaire (LVCP). Un guide semi-rigide conduit et contrôle le bon positionnement de l'implant inter-corporéal avant d'être retiré en retraversant le tube-guide introducteur.

Pour sa bonne compréhension, l'invention est à nouveau décrite ci-dessous en référence au dessin schématique annexé représentant, à titre d'exemples non limitatifs, plusieurs formes de réalisation possibles de l'ensemble qu'elle concerne.
La figure 1 est une vue de côté de deux vertèbres pathologiques destinées à être traitées par cet ensemble ;
la figure 2 est une vue en perspective d'un implant postérieur que comprend cet ensemble, selon une forme de réalisation ;
la figure 3 est une vue en perspective d'un implant inter-corporéal que peut comprendre cet ensemble, selon une forme de réalisation ;
les figures 4 et 5 sont des vues de dessus de cet implant inter-corporéal, respectivement en cours de son introduction entre les corps vertébraux, au moyen d'un instrument prévu à cet effet, et après mise en place entre ces corps vertébraux ;
la figure 6 est une vue de côté des deux vertèbres après mise en place des deux implants ;
la figure 7 est une vue similaire à la figure 5 d'un implant inter-corporéal selon une autre forme de réalisation ;
la figure 8 est une vue en coupe d'un implant inter-corporéal selon encore une autre forme de réalisation, et
la figure 9 est une vue d'un implant postérieur que comprend cet ensemble, selon une autre forme de réalisation, et de deux vertèbres sur lesquelles cet implant est destiné à être mis en place.

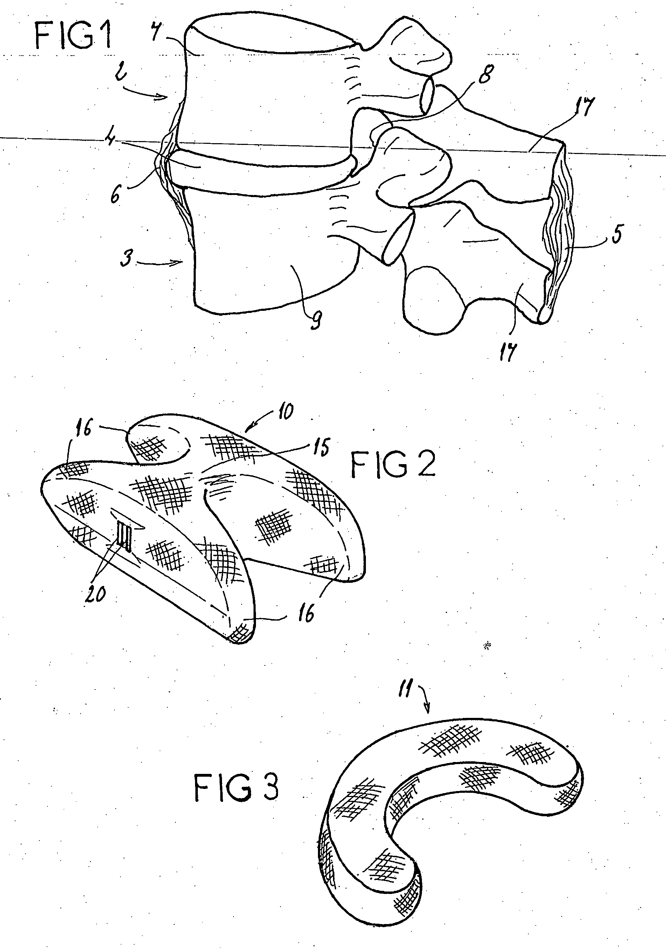

La figure 1 représente deux vertèbres 2, 3 dont le disque intervertébral 4 est affaissé. Cet affaissement conduit à une distension du ligament postérieur supra-épineux 5 et du ligament commun antérieur 6, à une sur-sollicitation des facettes 8 en pression, pouvant générer des lésions à celles-ci, et à un risque de contact des corps vertébraux 7, 9 l'un contre l'autre sur le côté antérieur.

Pour traiter cette pathologie, l'invention propose un ensemble de deux implants 10, 11, agissant conjointement, à savoir un implant postérieur 10, visible sur la figure 2, et un implant inter-corporéal 11, visible sur la figure 3.

L'implant postérieur 10 est formé par un noyau en silicone entouré d'une enveloppe tressée, notamment en fibres de polyester, assurant la protection de ce noyau. Il présente une portion inter-épineuse 15 et deux paires d'oreilles latérales 16 faisant saillie longitudinalement de part et d'autre de cette portion 15.

La portion 15 a une épaisseur légèrement supérieure à l'espace inter-épineux anatomique lorsque les vertèbres 2, 3 sont en lordose, de sorte qu'elle est légèrement comprimée par les apophyses épineuses 17 lorsque l'implant 10 est mis en place à la jonction lame-épineuse. L'implant 10 permet ainsi, dans cette position, de remettre le ligament postérieur supra-épineux 5 en tension anatomique.

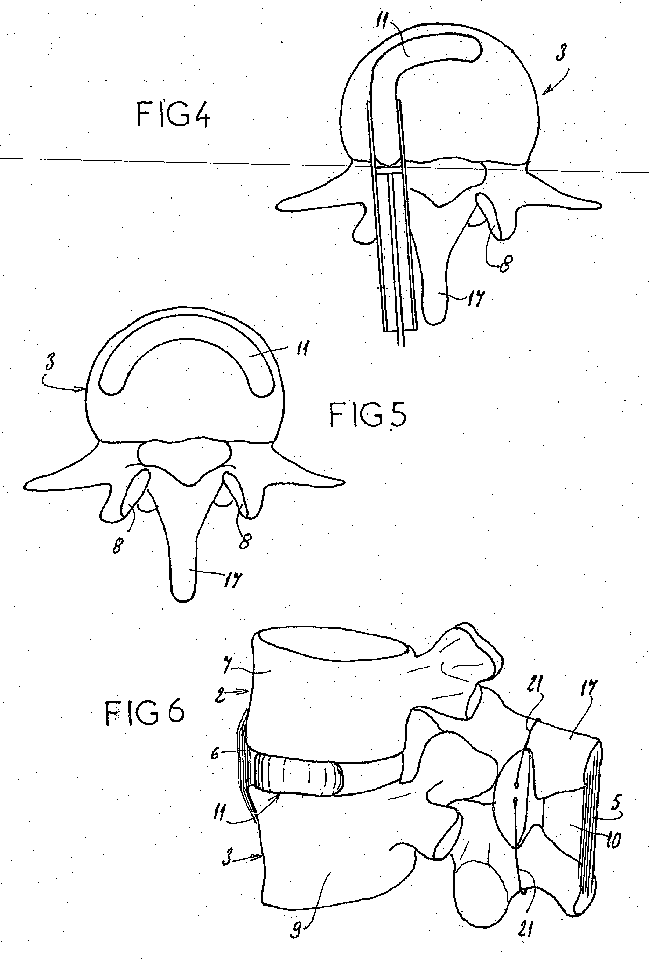

La portion 15 est percée de deux conduits transversaux 20 destinés à recevoir, ainsi que le montre la figure 6, deux liens 21 indépendants. Ces liens 21 servent à relier étroitement l'implant 10 aux apophyses 17. Chaque lien 21 peut être constitué par une tresse dont une extrémité est sertie sur l'extrémité d'une aiguille courbe d'insertion et dont l'autre extrémité comporte un anneau destiné à être serti sur le lien 21 après serrage étroit de ce lien sur l'apophyse 17 correspondante.

Les oreilles 16 ont des hauteurs importantes par rapport à la hauteur totale de l'implant 10, de l'ordre, pour les oreilles supérieures et inférieures, de 33 % et de 40 % de cette hauteur totale, respectivement. Les faces internes de deux oreilles 16 d'une même paire d'oreilles sont inclinées de manière à converger l'une vers l'autre en direction du fond de l'évidement qu'elles délimitent entre elles. Les oreilles 16 ont en outre une épaisseur moyenne relativement importante par rapport à la largeur moyenne de l'implant 10, de l'ordre, pour les oreilles supérieures et inférieures, de 27 % et de 30 % de cette largeur moyenne, respectivement.

Ces oreilles 16 permettent d'assurer le maintien de l'implant 10 entre les apophyses 17 nonobstant les mouvements relatifs des vertèbres 2, 3, en particulier les mouvements de pivotement selon l'axe de la colonne vertébrale.

L'implant inter-corporéal 11 est également formé par un noyau en silicone entouré d'une enveloppe tressée, notamment en fibres de polyester, assurant la protection de ce noyau. Il présente une forme courbe, en portion d'anneau, et est dimensionné de manière à s'étendre après mise en place le long d'une large portion antérieure des bords périphériques des corps vertébraux 7, 9. Il présente une hauteur telle qu'il permet, lorsqu'il est mis en place, de restaurer la hauteur anatomique du disque 4 et de remettre en tension anatomique le ligament commun antérieur 6.

En pratique, une ablation du complexe ligamentaire inter-épineux est réalisée par voie d'abord latéral puis les vertèbres 2, 3 sont distractées et l'implant postérieur 10 est mis en place entre les apophyses épineuses 17, immédiatement en arrière des facettes 8, c'est-à-dire à la jonction lame-épineuse. La souplesse des oreilles 16 ne fait pas obstacle à cette insertion. Les deux oreilles 16 se trouvant sur le côté par lequel l'implant 10 est inséré peuvent être maintenues en position rabattue l'une vers l'autre, pour faciliter l'insertion de l'implant 10.

Chaque lien 21 est ensuite engagé au travers de l'anneau qu'il comporte et est serré étroitement autour de l'apophyse 17 correspondante par coulissement au travers de cet anneau. L'anneau est alors serti sur le lien 21 de manière à maintenir ce lien en position de serrage de l'apophyse 17.

Ainsi maintenu, l'implant 10 est susceptible d'être sollicité tant en compression qu'en étirement.

L'implant 11 est quant à lui inséré entre les plateaux vertébraux des deux vertèbres 2, 3, par la même voie d'abord postéro-latérale que celle utilisée lors d'une dissectomie. Comme que le montre la figure 4, la mise en place de cet implant 11 est réalisé au moyen d'un instrument 25 comprenant un tube-introducteur 26 pourvu d'un piston 27 et d'une tige de piston 28. L'implant 11 est engagé, en étant comprimé, dans ce tube-introducteur et est expulsé de ce dernier au moment de sa mise en place, grâce au piston 27.

Les implants 10 et 11 permettent conjointement de rétablir l'écartement anatomique des vertèbres 2, 3 tant entre les facettes 8 qu'entre les plateaux vertébraux, mais également, et surtout, de conserver le ligament commun antérieur 6 et le ligament postérieur supra-épineux 5, en mettant ces ligaments en tension anatomique.

Lors d'une flexion de la colonne vertébrale vers l'avant, le ligament postérieur supra-épineux 5 peut alors à nouveau assurer sa fonction anatomique de limitation du mouvement. L'implant 10 permet, par son étirabilité, d'assister et de contrôler l'action de ce ligament. Simultanément, l'implant 11 restitue la fonction d'amortissement du disque 4 et absorbe les contraintes exercées en pression par le corps vertébral supérieur 7 sur le corps vertébral inférieur 9, en réalisant une assistance de l'*annulus fibrosus* périphérique. Il apporte ainsi d'amortissement progressif de ce corps vertébral supérieur 7, en prévenant tout risque de contact entre les corps vertébraux 7, 9.

Lors d'une flexion de la colonne vertébrale vers l'arrière, le ligament vertébral antérieur 6 peut à nouveau assurer sa fonction anatomique de limitation progressive du mouvement. Simultanément, l'implant 10 est comprimé et assiste alors les facettes 8 dans leur fonction de charnière et d'équilibrage postérieur.

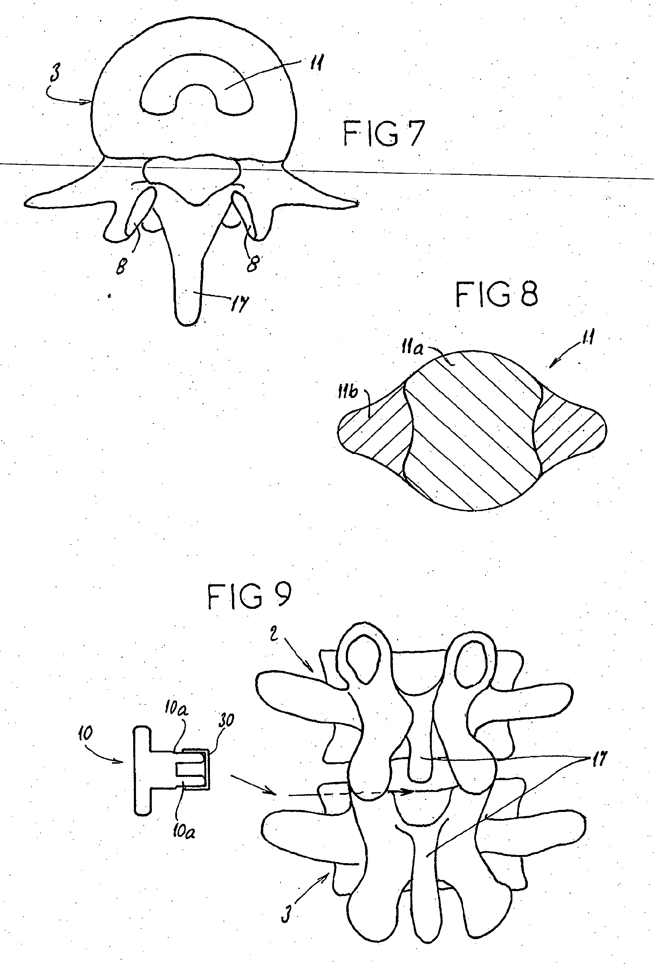

Les figures 7 ou 8 montrent que l'implant inter-corporéal 11 peut également, en remplacement ou en complément d'un implant inter-corporéal 11 tel que représenté sur les figures 1 à 6, être prévu pour remplacer et/ou assister le *nucléus.* Comme le montre la figure 7, il peut présenter alors une forme générale en "haricot" ou en "oméga", avec une portion médiane prolongée par deux lobes latéraux faisant saillie sur le côté postérieur, ou, comme le montre la figure 8, une forme en sphère pourvue d'un rebord équatorial réduisant son risque de déplacement. Dans ce deuxième cas, l'implant 11 comprend ladite sphère 11a et un anneau 11 b formant ledit rebord, l'ouverture de cet anneau 11 b ayant un diamètre inférieur à celui de la sphère 11a et cette sphère 11a étant engagée avec déformation au travers de cette ouverture puis étant fixée à l'anneau 11 b.

La figure 9 montre que l'implant postérieur 10 peut avoir une forme en "H" et comporter un "clip" 30 venant maintenir deux oreilles latérales 10a de cet implant dans un état déformé dans lequel ces oreilles 10a sont rapprochées l'une de l'autre. Le clip 30 permet ainsi une insertion latérale facilitée de l'implant 10 entre les apophyses épineuses 17 des vertèbres 2, 3 traitées, jusqu'à venue des oreilles non déformées contre les apophyses 17 puis est retiré ensuite pour déployer les oreilles 10a et maintenir ainsi l'implant en position.

Il apparaît de ce qui précède que l'invention apporte une amélioration déterminante à la technique antérieure, en fournissant un ensemble qui permet une stabilisation parfaitement fonctionnelle de vertèbres subissant à la fois une dégénérescence discale et une distension ligamentaire, ainsi qu'éventuellement une usure des facettes, et ce tout en étant relativement simple à implanter et relativement peu invasif. Cet ensemble forme ainsi une prothèse d'assistance à effet couplé.

Il va de soi que l'invention n'est pas limitée à la forme de réalisation décrite ci-dessus à titre d'exemple mais qu'elle en embrasse au contraire toutes les variantes de réalisation entrant dans le champ de protection défini par les revendications ci-annexées.

## Revendications

1. Ensemble de stabilisation de vertèbres lombaires (2, 3), comprenant :
- un implant postérieur (10) amortissant positionnable, par voie latérale, à la jonction lame-épineuse des deux vertèbres adjacentes (2, 3) traitées sans résection du ligament postérieur supra-épineux (5), ledit implant postérieur (10) présentant une hauteur telle qu'il permet, lorsqu'il est mis en place, de remettre le ligament postérieur supra-épineux (5) en tension anatomique ; et
- au moins un implant inter-corporéal (11) amortissant insérable entre les plateaux vertébraux desdites deux vertèbres adjacentes (2, 3), par la même voie postéro-latérale que celle utilisée lors d'une discectomie, ledit implant inter-corporéal (11) présentant une hauteur telle qu'il permet, lorsqu'il est mis en place, de restaurer la hauteur anatomique du disque intervertébral (4) et de remettre le ligament commun antérieur (6) en tension anatomique.

2. Ensemble selon la revendication 1, comprenant en outre des moyens de maintien (16, 21) pour assurer la continuité de l'implant postérieur (10) en position, avec les apophyses épineuses (17).

3. Ensemble selon la revendication 2, où les moyens de maintien comprennent une conformation appropriée de l'implant postérieur (10), définissant des évidements opposés de réception des apophyses épineuses (17).

4. Ensemble selon la revendication 2 ou revendication 3, où les moyens de maintien comprennent des moyens de fixation de l'implant postérieur (10) aux apophyses épineuses (17), tels que deux liens indépendants (21) ou des pièces rigides d'ancrage ne limitant pas la déformabilité de l'implant (10).

5. Ensemble selon l'une des revendications 1 à 4, où l'implant postérieur (10) est constitué en une seule pièce et est inséré entre les apophyses épineuses (17) des vertèbres (2, 3).

6. Ensemble selon l'une des revendications 3 à 5, où l'implant postérieur a une forme "en diabolo" ou en "H" et est en une seule pièce, et l'ensemble comprend une pièce venant maintenir cet implant postérieur dans un état déformé dans lequel deux oreilles latérales que comprend l'implant sont rapprochées l'une de l'autre, de manière à permettre une insertion latérale de l'implant entre les apophyses épineuses des vertèbres traitées.

7. Ensemble selon l'une des revendications 1 à 4, où l'implant postérieur est en deux parties assemblables, amenées chacune par un côté de l'espace inter-épineux et assemblées l'une à l'autre au niveau de cet espace.

8. Ensemble selon l'une des revendications 1 à 7, où l'implant inter-corporéal (11) est disposé, lorsqu'il est mis en place, de manière à s'étendre au niveau des zones latérales antérieures des plateaux vertébraux, pour renforcer la stabilité latérale des vertèbres (2, 3) et de permettre une assistance de *Vannulus fibrosus* périphérique.

9. Ensemble selon la revendication 8, où l'implant inter-corporéal (11) présente une forme courbe, en portion d'anneau.

10. Ensemble selon l'une des revendications 1 à 8, où l'implant inter-corporéal sert à remplacer et/ou assister le *nucléus.*

11. Ensemble selon la revendication 10, où l'implant inter-corporéal présente une forme générale en "haricot" ou en "oméga", avec une portion médiane prolongée par deux lobes latéraux faisant saillie sur le côté postérieur, ou en sphère.

12. Ensemble selon l'une des revendications 1 à 11, où le ou un implant inter-corporéal a une section transversale triangulaire ou trapézoïdale pour implantation avec son plus grand côté latéral tourné vers le côté antérieur.

13. Ensemble selon l'une des revendications 1 à 12, où le ou un implant inter-corporéal (11) comprend des moyens pour assurer son maintien entre les plateaux vertébraux.

14. Ensemble selon la revendication 13, où les moyens de maintien de l'implant inter-corporéal (11) entre les plateaux vertébraux comprennent une conformation de l'implant inter-corporéal (11) adaptée à la forme des plateaux vertébraux, de manière à maintenir cet implant inter-corporéal (11) entre les vertèbres (2, 3).

15. Ensemble selon l'une des revendications 1 à 14, où l'implant postérieur (10) et/ou l'implant inter-corporéal (11) comprend un noyau en matériau amortissant, tel qu'une silicone, un polyuréthane, un polymère hydrophile, un polycarbonate, ou une pièce en métal à mémoire de forme, et une enveloppe entourant ce noyau.

16. Combinaison d'un implant inter-corporéal (11) selon l'une des revendications 1 à 15 et d'un instrument pour positionner l'implant inter-corporéal, l'instrument comprenant un tube-guide (26) introducteur pourvu d'un piston (27), l'implant (11) étant engageable, en étant comprimé, dans ce tube-introducteur (26) et pour l'expulsion de ce dernier au moyen du piston (27).

## Patentansprüche

1. Stabilisierungseinheit für Lendenwirbel (2, 3), umfassend:
- ein dämpfend positionierbares, rückseitiges Implantat (10) auf seitlichem Weg, an der Wirbelverbindung von zwei anliegenden Wirbeln (2, 3) behandelt ohne Resektion der hinteren Supraspinatussehne (5), wobei das rückseitige Implantat (10) eine solche Höhe aufweist, dass damit nach dem Einsetzen die hintere Supraspinatussehne (5) in anatomische Spannung versetzt werden kann, und
- mindestens ein interkorporelles Implantat (11) dämpfend einsetzbar zwischen den Endplatten der beiden anliegenden Wirbel (2, 3) auf demselben posterolateralen Weg wie der, der bei einer Disektomie verwendet wird, wobei das interkorporelle Implantat (11) eine solche Höhe aufweist, dass damit nach dem Einsetzen die anatomische Höhe der Bandscheibe (4) wiederhergestellt und das vordere gemeinsame Band (6) in anatomische Spannung versetzt werden kann.

2. Einheit nach Anspruch 1, umfassend u. a. Haltemittel (16, 21) zum Gewährleisten der Beibehaltung der Position des rückseitigen Implantats (10) mit den Wirbelfortsätzen (17).

3. Einheit nach Anspruch 2, wobei die Haltemittel eine geeignete Form des rückseitigen Implantats (10) umfassen, die gegenüberliegende Aussparungen zur Aufnahme der Wirbelfortsätze (17) definieren.

4. Einheit nach Anspruch 2 oder Anspruch 3, wobei die Haltemittel Befestigungsmittel des rückseitigen Implantats (10) an den Wirbelfortsätzen (17) umfassen, so dass zwei unabhängige Bindeglieder (21) oder starre Verankerungsteile nicht die Verformbarkeit des Implantats (10) beschränken.

5. Einheit nach einem der Ansprüche 1 bis 4, wobei das rückseitige Implantat (10) aus einem einzigen Teil besteht und zwischen den Wirbelfortsätzen (17) der Wirbel (2, 3) eingesetzt wird.

6. Einheit nach einem der Ansprüche 3 bis 5, wobei das rückseitige Implantat die Form eines "Diabolos" oder eines "H" hat, und die Einheit über ein Teil verfügt, die dieses rückseitige Implantat in einem solchen Verformungszustand hält, in dem zwei seitliche Ohren, die das Implantat umfasst, so aneinander angenähert werden, dass sie ein seitliches Einsetzen des Implantats zwischen die Wirbelfortsätze der behandelten Wirbel ermöglichen.

7. Einheit nach einem der Ansprüche 1 bis 4, wobei das rückseitige Implantat aus zwei zusammensetzbaren Teilen besteht, die jeweils auf einer Seite des Wirbelzwischenraums angeordnet und miteinander auf Höhe dieses Raums zusammengesetzt werden.

8. Einheit nach einem der Ansprüche 1 bis 7, wobei das interkorporelle Implantat (11) beim Anbringen so angeordnet wird, dass es sich auf der Ebene seitlicher Vorderseiten der Endplatten erstreckt, um die seitliche Stabilität des Wirbels (2, 3) zu verstärken und eine Unterstützung des kugelförmigen *Anulus Fibrosus* zu ermöglichen.

9. Einheit nach Anspruch 8, wobei das interkorporelle Implantat (11) eine gebogene Ringform aufweist.

10. Einheit nach einem der Ansprüche 1 bis 8, wobei das interkorporelle Implantat als Ersatz und/oder zur Unterstützung des Nucleus dient.

11. Einheit nach Anspruch 10, wobei das interkorporelle Implantat generell "bohnenförmig" oder "omegaförmig" ist und der mittlere Abschnitt durch zwei seitliche Lappen verlängert ist, die auf der Rückseite hervorstehen, oder kugelförmig ist.

12. Einheit nach einem der Ansprüche 1 bis 11, wobei das oder ein interkorporelles Implantat einen dreieckigen oder trapezförmigen Querschnitt hat, der mit seiner größten Seite seitlich zur Vorderseite gedreht eingesetzt wird.

13. Einheit nach einem der Ansprüche 1 bis 12, wobei das oder ein interkorporelles Implantat (11) die Mittel umfasst, um seine Halterung zwischen den Endplatten zu gewährleisten.

14. Einheit nach Anspruch 13, wobei die Haltemittel des interkorporellen Implantats (11) zwischen den Endplatten eine Form des interkorporellen Implantats (11) umfassen, die an die Form der Endplatten angepasst ist, damit dieses interkorporelle Implantat (11) zwischen den Wirbeln (2, 3) gehalten wird.

15. Einheit nach einem der Ansprüche 1 bis 14, wobei das rückseitige Implantat (10) und/oder das interkorporelle Implantat (11) einen Kern aus dämpfendem Material, wie z. B. ein Silikon, ein Polyurethan, ein hydrophiles Polymer, ein Polykarbonat oder ein Teil aus Metall mit Formgedächtnis, und eine Hülle um diesen Kern umfasst.

16. Kombination aus einem interkorporellen Implantat (11) nach einem der Ansprüche 1 bis 15 und einem Instrument zum Positionieren des interkorporellen Implantats, wobei das Instrument ein einführendes Führungsrohr (26) mit einem Kolben (27) umfasst, und das Implantat (11) in komprimiertem Zustand in dieses Einführungsrohr (26) eingesetzt und mittels des Kolbens (27) herausgepresst werden kann.

## Claims

1. An assembly for stabilising lumbar vertebrae (2, 3), comprising:
a shock-absorbent posterior implant (10) positionable, via the lateral route, at the lamina/spinous process junction of the two adjacent vertebrae (2, 3) being treated, without resection of the supra-spinous posterior ligament (5), said posterior implant (10) having a height such that, when it is positioned, it allows the supra-spinous posterior ligament (5) to be reset at anatomical tension; and
at least one shock-absorbent inter-corporeal implant (11) insertable between the vertebral plates of said two adjacent vertebrae (2, 3) via the same posterior/lateral route as that used during a dissectomy, said inter-corporeal implant (11) having a height such that, when it is positioned, it allows the anatomical height of the intervertebral disc (4) to be restored and the anterior common ligament (6) to be reset at anatomical tension.

2. An assembly according to claim 1, further comprising securing means (16, 21) in order to ensure the continuity of the posterior implant (10) in position, relative to the spinous processes (17).

3. An assembly according to claim 2, wherein the securing means comprise a suitable form of the posterior implant (10), defining opposed recesses for receiving the spinous processes (17).

4. An assembly according to claim 2 or claim 3, wherein the securing means comprise means for fixing the posterior implant (10) to the spinous processes (17), such as two independent cords (21) or rigid anchoring pieces, which do not limit the deformability of the implant (10).

5. An assembly according to anyone of claims 1 to 4, wherein the posterior implant (10) is constituted as a single piece and is inserted between the spinous processes (17) of the vertebrae (2, 3).

6. An assembly according to anyone of claims 3 to 5, wherein the posterior implant is of a "diabolo" or "H"-like shape and is in a single piece, and the assembly comprises a piece which keeps this posterior implant in a deformed state, in which two lateral lugs, which the implant comprises, are brought closer towards each other, in order to allow lateral insertion of the implant between the spinous processes of the vertebrae being treated.

7. An assembly according to anyone of claims 1 to 4, wherein the posterior implant is in the form of two parts which can be assembled together and which are each brought at one side of the inter-spinous space and are assembled together in this space.

8. An assembly according to anyone of claims 1 to 7, wherein the inter-corporeal implant (11) is arranged, when positioned, to extend in the anterior lateral zones of the vertebral plates to reinforce the lateral stability of the vertebrae (2, 3) and to allow relief of the peripheral *annulus fibrosus.*

9. An assembly according to claim 8, wherein the inter-corporeal implant (11) has a curved shape, in the form of a portion of a ring.

10. An assembly according to anyone of claims 1 to 8, wherein the inter-corporeal implant is to replace and/or assist the *nucleus.*

11. An assembly according to claim 10, wherein the inter-corporeal is of a general "bean" or "omega"-like shape, with a central portion which is extended by two lateral lobes protruding at the posterior side, or in the form of a sphere.

12. An assembly according to anyone of claims 1 to 11, wherein the or a inter-corporeal implant has a triangular or trapezoidal cross-section for implantation with the largest lateral side thereof directed towards the anterior side.

13. An assembly according to anyone of claims 1 to 12, wherein the or a inter-corporeal implant (11) comprises means for ensuring the securing thereof between the vertebral plates.

14. An assembly according to claim 13, wherein the means for securing the inter-corporeal implant (11) between the vertebral plates comprise a form of the inter-corporeal implant (11), which form is adapted to the shape of the vertebral plates in order to secure this inter-corporeal implant (11) between the vertebrae (2, 3).

15. An assembly according to anyone of claims 1 to 14, wherein the posterior implant (10) and/or the inter-corporeal implant (11) comprise(s) a core of shock-absorbent material, such as a silicone, a polyurethane, a hydrophilic polymer, a polycarbonate, or a piece of shape-memory metal, and a casing which surrounds the core.

16. A combination of an inter-corporeal implant (11) according to anyone of claims 1 to 15 and an instrument for positioning the inter-corporeal implant, the instrument comprising an introduction guide tube (26) which is provided with a piston (27), the implant (11) being engageable, with compression, in the introduction tube (26) and for expulsion therefrom by means of the piston (27).
